# EUROPEAN PATENT SPECIFICATION

(11) **EP 1 404 306 B1**
(45) Date of publication and mention of the grant of the patent: **20.10.2010**
(21) Application number: 02744014.8
(22) Date of filing: 14.06.2002
(51) Int. Cl.: A61K 9/68, A61K 31/465, A61P 43/00

(54) **A COATED NICOTINE-CONTAINING CHEWING GUM, MANUFACTURE AND USE THEREOF**
BESCHICHTETER NIKOTINHALTIGER KAUGUMMI, SEINE HERSTELLUNG UND VERWENDUNG
GOMME A MACHER ENROBEE CONTENANT DE LA NICOTINE, SA FABRICATION ET SON UTILISATION

(30) Priority: 20.06.2001 SE 0102197
(43) Date of publication of application: 07.04.2004
(73) Proprietor: McNeil AB, 254 42 Helsingborg (SE)
(72) Inventor: LINDELL, Katarina, S-241 93 eslöv (SE); LINDBERG, Nils-Olof, S-216 15 Malmö (SE); OLSSON, Roland, SE-253 73 Helsingborg (SE)
(74) Representative: Hedenström, John Allan
(86) International application number: PCT/SE2002/001158
(87) International publication number: WO 2002/102357

(56) References cited:
- EP-A2- 0 745 380
- WO-A1-00/19977
- WO-A1-00/35296
- WO-A1-00/56281
- WO-A2-00/13662
- GB-A- 2 289 204
- US-A- 5 549 906
- US-A- 5 972 974

## Description

### Technical Field

This invention relates to a coated chewing gum product for delivering nicotine to a subject. Also contemplated is a method and a system for delivering nicotine as well as use and production of said coated chewing gum product.

### Background of the Invention

### Tobacco dependence and reduction thereof

In recent years, with the recognition of the harmful effects of tobacco smoking, there have been numerous campaigns and programs by governmental agencies and various health groups and other interested organisations to disseminate information about the adverse health effects resulting from tobacco smoking. Moreover, and as a result of this recognition of the harmful effects, there have been many programs directed to attempts in reducing smoking incidence.

Nicotine is an organic compound and is the principal alkaloid of tobacco. Nicotine is the chief addictive ingredient in the tobacco used in cigarettes, cigars, snuff and the like. Nicotine is also an addictive drug, though, and smokers characteristically display a strong tendency to relapse after having successfully stopped smoking for a time. Nicotine is the worlds second most used drug, after caffeine from coffee and tea.

The main problem with tobacco smoking is its enormous implications on health. Today it is estimated that smoking related diseases cause some 3 - 4 million deaths per year. In the US Surgeon General's 1988 report on The Health Consequences of Smoking, it was estimated that in the US alone about 300.000 deaths are caused each year by diseases related to cigarette smoking. In fact, excessive smoking is now recognised as one of the major health problems throughout the world. This grim consequence of tobacco smoking has urged many medical associations and health authorities to take very strong actions against the use of tobacco.

Even though tobacco smoking is decreasing in many developed countries today it is hard to see how the societies could get rid of the world's second most used drug.

The most advantageous thing a heavy smoker can do is to reduce or preferably even stop smoking completely. Experience shows, however, that most smokers find this extremely difficult since, mostly, tobacco smoking results in a dependence disorder or craving. The WHO has in its International Classification of Disorders a diagnosis called Tobacco Dependence. Others like the American Psychiatric Association call the addiction Nicotine Dependence. It is generally accepted that these difficulties to stop smoking result from the fact those heavy smokers are dependent on nicotine. The most important risk factors are, however, substances that are formed during the combustion of tobacco, such as carbon monoxide, tar products, aldehydes, and hydrocyanic acid.

### Effects of nicotine

The administration of nicotine can give satisfaction and the usual method is by smoking, either by smoking e g a cigarette, a cigar or a pipe. However, smoking has health hazards and it is therefore desirable to formulate an alternative way of administering nicotine in a pleasurable manner that can be used to facilitate withdrawal from smoking and/or used as a replacement for smoking.

When smoking a cigarette, nicotine is quickly absorbed into the smoker's blood and reaches the brain within around ten seconds after inhalation. The quick uptake of nicotine gives the consumer a rapid satisfaction, or kick. The satisfaction, then, lasts during the smoking time of the cigarette and for a period of time thereafter. The poisonous, toxic, carcinogenic, and addictive nature of smoking has provided efforts for methods, compositions and devices, which help in breaking the habit of smoking cigarettes.

Nicotine is an addictive poisonous alkaloid C₅H₄NC₄H₇NCH₃, derived from the tobacco plant. Nicotine is also used as an insecticide. Approximately forty milligrams of nicotine is able to kill an adult (Merck Index).

### Nicotine replacement products

One way to reduce smoking is to provide nicotine in a form or manner other than by smoking and some products have been developed to fulfil this need. Nicotine containing formulations are currently the dominating treatments for tobacco dependence.

The successes in achieving reduction in the incidence of smoking have been relatively poor using presently known products. The present state of the art involves both behavioural approaches and pharmacological approaches. More than 80 % of the tobacco smokers who initially quit smoking after using some behavioural or pharmacological approach to singly reduce smoking incidence generally relapse and return to the habit of smoking at their former rate of smoking within about a one year's period of time.

As an aid for those who are willing to stop smoking there are several ways and forms of nicotine replacement products available on the market; such as nicotine chewing gum. Several methods and means have been described for diminishing the desire of a subject to use tobacco, which comprises the step of administering to the subject nicotine or a derivative thereof as described in e.g US 5 810 018 (oral nicotine spray), US 5 939 100 (nicotine containing microspheres) and US 4 967 773 (nicotine containing lozenge).

Nicotine-containing nose drops have been reported (Russell et al., British Medical Journal, Vol. 286, p. 683 (1983); Jarvis et al., Brit. J. of Addiction, Vol. 82, p. 983 (1987)). Nose drops, however, are difficult to administer and are not convenient for use at work or in other public situations. Ways of administrating nicotine by way of delivering directly into the nasal cavity by spraying is known from US 4 579 858, DE 32 41437 and WO/93 127 64. There may, though, be local nasal irritation with use of nasal nicotine formulations. The difficulty in administration also results in unpredictability of the dose of nicotine administered.

The use of skin patches for transdermal administration of nicotine has been reported (Rose, in Pharmacologic Treatment of Tobacco Dependence, (1986) pp. 158-166, Harvard Univ. Press). Nicotine-containing skin patches that are in wide use today can cause local irritation and the absorption of nicotine is slow and affected by cutaneous blood flow.

Also, inhaling devices resembling a cigarette are known for uptake of nicotine vapours as suggested in US 5 167 242. Said means and methods address the problems associated with addiction to nicotine.

### Nicotine chewing gum

One of the most successful approaches to date in reducing the incidence of smoking relies upon nicotine containing chewing gum that is designed to reduce smoking withdrawal symptoms. The reported success rate is approximately twice that of placebo. The use of the nicotine gum suffers from several problems e g that it has been found that the nicotine containing gum does not sufficiently rapidly satisfy the craving that most smokers experience.

### Prior art and problems thereof

One successful product that is used as a smoking substitute and/or as a smoking cessation aid and which is based on nicotine, is the chewing gum Nicorette^{®}. This product was one of the first nicotine replacement forms that was approved by the Food and Drug Administration (FDA) and is still one of the most used nicotine replacement products. Nicorette^{®} chewing gum has been on the market in about 60 countries for several years. In this chewing gum the nicotine is present in the form of a complex with an insoluble cation-exchanger (polacrilex) that is dispersed in a gum base. The nicotine is slowly released from the gum due to chewing and will reach similar plasma levels as when smoking a cigarette after about 30 minutes depending on the chewing technique, i e slow or active. Patents related to this product are, e g, the US Patents 3,877,468, 3,901,248 and 3,845,217.

WO 98/23165 discloses a chewing gum wherein nicotine may be in the coating. This concept may provide rapid release of the nicotine from the coated chewing gum, but not a sufficiently rapid buccal uptake of the nicotine. The fraction of the released nicotine that is not immediately absorbed will be flushed down in the gastrointestinal (G.I.) tracts by the saliva, thereby possibly causing hiccups and other G.I. side effects. Once absorbed by the G.I. route this swallowed nicotine will be subjected to first pass metabolism.

WO 00/13662 discloses a chewing gum for systemic, oral administration of an active whereby said active is administered by the chewing gum composition in a bi-phasic manner. The bi-phasic delivery is obtained by the gum matrix as such, not from any coating.

WO 00/19977 discloses a substantially moisture free and possibly coated chewing gum for delivery of an active. The possible coating is not said to be buffered.

WO 00735296 discloses a chewing gum having a coating, which may contain nicotine, but which is devoid of any buffer.

WO 00/561281 discloses a chewing gum comprising nicotine and β-cyclodextrin.

GB 2289204 discloses a chewing gum comprising a capsacinoid.

EP 075380 discloses a nicotine-containing lollipop, which may be used for alleviating symptoms of Alzheimer's disease, ulcerative colitis, Tourette's syndrome and Parkinson's disease.

It is highly desirable in light of the aforementioned problems to develop means and methods for the administration of nicotine to give a satisfaction to a person craving for nicotine or to provide a sense of smoking satisfaction without smoking, which can also avoid problems associated with the prior art means and methods. In this respect, the present invention addresses this need and interest.

### Summary of the Invention

In view of the foregoing disadvantages known in the art when trying to deliver nicotine to a subject so as to obtain a rapid transmucosal uptake of nicotine in the oral cavity of the subject the present invention provides a new and improved product, systems and methods for obtaining a rapid transmucosal uptake of nicotine in the oral cavity of the subject. The invention is as defined in the claims.

An object of the present invention is to provide an efficient and effective product, as well as methods and systems for a rapid uptake of nicotine in a subject to avoid the disadvantages of such previously known products and methods.

Thus, the present invention provides a method for delivering nicotine in any form to a subject comprising administering to a subject said coated chewing gum product containing nicotine in any form into the oral cavity of the subject and allowing the nicotine in any form in the coated chewing gum product to be released in the saliva in the oral cavity and absorbed into the systemic circulation of the subject as well as a method for producing said coated chewing gum.

The present invention also provides a method for obtaining reduction of the urge to smoke or use tobacco containing material and/or for providing a sense of smoking satisfaction without smoking, comprising the steps of replacing at least partly the tobacco containing material with above said coated chewing gum product, administering to a subject a coated chewing gum product containing nicotine in any form into the oral cavity of the subject and allowing the nicotine in any form of the coated chewing gum product to be released in the saliva in the oral cavity and absorbed by the subject.

Furthermore, the present invention provides a system for delivering nicotine in any form to a subject, comprising said coated chewing gum product and at least one other means for obtaining reduction of the urge to smoke or use of tobacco as well as a system for obtaining reduction of the urge to smoke or otherwise use of tobacco and/or for providing a sense of smoking satisfaction without smoking, comprising a coated chewing gum product according to above and at least one other method for obtaining reduction of the urge to smoke or otherwise use of tobacco. Said system may be a system wherein the at least other method is selected from the group consisting of administration through mouth sprays, nasal sprays, transdermal patches, inhaling devices, lozenges, tablets and parenteral methods, subcutaneous methods, intravenous methods, rectal methods, vaginal methods and transmucousal methods; or otherwise use of tobacco.

Still furthermore the present invention relates to a coated chewing gum product comprising at least one chewing gum core, nicotine in any form and/or a nicotine mimicking agent, at least one coating layer and optionally at least one or more other additive, wherein said at least one coating layer is buffered.

Said product may further comprise at least one core being buffered and further may also the nicotine in any form be a part of the at least one coating layer or at least one of the at least one coating layers. The at least one coating is buffered, according to the invention, in such a way that upon administration of the gum the pH of the saliva is increased by 0.3 - 4 pH units, or preferably increased by 0.5 - 2 pH units. Said product is buffered by the use of a buffer selected from the group consisting of a carbonate, including bicarbonate or sesquicarbonate, glycinate, phosphate, glycerophosphate or citrate of an alkali metal, such as potassium and sodium, e g trisodium and tripotassium citrate, or ammonium, and mixtures thereof.

Use of said product will according to the invention rapidly deliver nicotine in any form to a subject and will also be used for obtaining a quick and/or sustained and/or complete reduction of the urge to smoke or use tobacco and/or for providing a sense of smoking satisfaction without smoking resembling the sense of smoking satisfaction and reduction of the urge to smoke obtained after regular smoking or use of tobacco.

### Detailed Description of the Invention

### Definitions

As used herein, the term "chewing gum product" intends to mean all chewable gum products.

The term "fast reduction of the urge to smoke or use tobacco " is herein intended to mean an initial priming of the subject so as to achieve a reduction of the urge to smoke or use tobacco.

The term "sustained" is herein intended to mean prolonged over time.

The term "complete reduction " or "complete" is herein intended to mean complete or substantially complete reduction.

The term "controlled release" is intended to mean a release of a substance from a gum by the aid of active chewing of the gum in the oral cavity of the subject, whereby the active chewing is controlling the amount of substance released.

The term "slow release " is intended to mean that the nicotine is released from the gum upon, e g chewing, over a period of time e g several minutes to an hour.

The term "unit formula" is intended to mean one chewing gum product.

The term "transient" is intended to mean a non-permanent change, upon which the relevant state, e g biological or physiological state, after a certain period of time will return to its value or behaviour prior to said change.

The term "buccal" and "buccally" are herein intended to pertain to all of or any part of the tissue of the oral cavity.

### The coated chewing gum

Presently existing nicotine chewing gums provide a slow release and a slow uptake of nicotine. This will not resemble the actual sense of satisfaction when smoking, where an initial fast uptake of nicotine is achieved giving the smoker, i e the subject, a sense of satisfaction. Accordingly, as revealed above, the present invention relates to a coated chewing gum product for improving the absorption of nicotine in a subject, and wherein the absorption is quicker than by using current means and methods known in the art of nicotine chewing gums. Such a rapid transmucosal uptake of the nicotine in the oral cavity is expected to give more of a cigarette like sense of satisfaction and a more rapid reduction of the urge to smoke and use tobacco.

According to the invention, a coated chewing gum product is used for improving the absorption of said nicotine. The coated chewing gum product comprises at least one chewing gum core, nicotine in any form and/or a nicotine mimicking agent, at least one coating layer and at least one other additive, wherein at least one of said coating layer is buffered.

Also, the at least one core may be buffered in different embodiments. The core may be buffered with the same or different ways of buffering as the at least one coating layer.

Said buffering of the at least one coating layer and optionally the at least one core generates a coated chewing gum product giving improved absorption kinetics of nicotine compared to in the art known chewing gum products.

The chewing gum product may be a medicated chewing gum. Medicated chewing gums are herein intended to mean solid or semi-solid, single-dose preparations with a base consisting mainly of gum that are intended to be chewed but not swallowed, where the chewing gums act as a drug delivery system. They contain one or more active substances, which are released by chewing. After dissolution or dispersion of the active substance in the saliva, such chewing gums are used for i) local treatment of mouth diseases and ii) systemic delivery after absorption through transmucosal uptake throughout the oral cavity.

### The buffering agent

Absorption of nicotine from the oral cavity to the systemic circulation is dependent on the pH of the saliva, pH of the blood plasma and the pKa of nicotine, which is about 7.8. Assuming a pH of the plasma of 7.4 and of the saliva of 6.8 only about 10% of the nicotine will be in the free base form. Thus, in order to promote absorption of nicotine in a free base form, which is the form predominantly absorbed through the mucosa, the pH of the saliva must be increased. At a pH of 8.8 about 90% of the nicotine will then be in the free base form.

Thus according to the invention, the coated chewing gum product is buffered. This may be achieved by including physiologically acceptable buffering substances or agents, or by other means. With other means it is intended to include buffering by any component in the product, which may not normally act as a buffering agent, such as a self-buffering additive or gum base.

According to the invention, at least one coating layer is buffered. In specific embodiments, also the at least one core is buffered.

In specific embodiments, the at least one coating layer is buffered in such a way that upon administration of the gum the pH of the saliva is increased 0.3 - 4 pH units, preferably 0.5 - 2 pH units. The buffering is designed so as to achieve a transient buffering of the saliva of a subject during melting, disintegration or dissolution of the coating layer or layers. As the change is transient, the pH will return to its normal value after a certain period of time.

Similarly, the at least one core may be buffered. This may allow said change in the pH to be ensured during chewing of the core of the gum product, where the chewing allows the suitable buffer agent or substance or other means to produce a transient change in the pH of the saliva, e g an increase in the pH.

By employing the said change, here as an increase, in said pH of the saliva the transmucosal uptake of nicotine in the oral cavity is changed, e g increased compared to the nicotine uptake when the saliva is not buffered according to the invention. Also, since the transmucosal uptake of nicotine in the oral cavity according to the invention is faster than for nicotine not being buffered according to the invention, less nicotine will be swallowed to reach the gastrointestinal (G.I.) tract. The nicotine that reaches the G.I. tract will be subjected to first pass metabolism which reduces the total amount of intact nicotine absorbed. This means that the bio-availability of nicotine that is not co-administered with a buffer according to the invention will generally be lower than when administered together with a buffer.

Further embodiments of the invention includes combinations wherein the at least one coating layer is buffered by the use of a buffer selected from the group consisting of a carbonate including bicarbonate or sesquicarbonate, glycinate, phosphate, glycerophosphate or citrate of an alkali metal, such as potassium or sodium, or ammonium, and mixtures thereof.

Further embodiments may use trisodium or tripotassium citrate, and mixtures thereof.

Still further embodiments may use different phosphate systems, such as trisodium phosphate, disodium hydrogen phosphate; and tripotassium phosphate, dipotassium hydrogen phosphate, and calcium hydroxide, sodium glycinate; and mixtures thereof.

Alkali metal carbonates, glycinates and phosphates are preferred buffering agents.

The one or more buffering agents may to some extent be microencapsulated or otherwise coated as granules with polymers and/or lipids being less soluble in saliva than is the one or more buffering agents. Such microencapsulation controls the dissolution rate whereby is extended the time frame of the buffering effect.

In order to increase the buffering capacity still further without correspondingly increasing the pH, one may in specific embodiments use a second or auxiliary buffering agent to the first buffering agent, such as e g sodium or potassium bicarbonate buffers. The second or auxiliary buffering agent may be selected from the group consisting of alkali metal bicarbonates that are preferred for this purpose. Thus, further embodiments of the invention may comprise a mixture of an alkali metal carbonate or phosphate and alkali metal bicarbonate.

The amount of the buffering agent or agents in the chewing gum composition is preferably sufficient in the specific embodiments to raise the pH of the saliva to above 7, as specified above, to transiently maintain the pH of the saliva in the oral cavity above 7, e g pH7-11.

The nicotine may be administered in different forms, e g in different complexes or salts. The amount of buffer required to achieve such an increase in pH of the different administered nicotine form is readily calculated by the skilled man in the art. The extent and duration of the increase in pH is dependent on type and amount of the buffering agent(s) used as well as where, i e in the at least one coating layer and optionally in the at least one core, the buffer is distributed in the product and is further described within the paragraphs below.

### The coating of the gum core

The process of coating a chewing gum is well known in the art. The present invention provides a coating, to facilitate the uptake of administered nicotine in any form to the subject. Known intentions of coating a chewing gum product may be to add crispiness, enhance taste, or to protect the gum, e g during storage, or as to tone down bad or irritating taste of the gum product.

According to the invention, the chewing gum is a coated chewing gum comprising at least one coating layer.

The different embodiments according to the invention may use sugar coating, film coating, press/compression coating or melt coating.

For the film and sugar coating, the coating procedure may be manual or the coating may be sprayed onto the gum core/pellet in rotating pans of different shapes or fluidised beds in combination with evaporation of the solvent, e g water or organic solvent.

Sugar coating is a multistep process and may be divided into the following steps:
1. sealing of the cores
2. subcoating
3. smoothing, or glossing
4. colouring
5. polishing
6. optionally printing

Sugar coated cores have a smoother profile with less visible edges remaining from the original core. Sub-coating, e g either by dusting with powder on the sucrose solution or application of dry powder in the sucrose solution, may be used. The core may be sugar coated by a panning technique, e g using a sugar coating pan, or other more sophisticated techniques capable of some degree of automation.

The sugar in a sugar coating may not be just sucrose. Also other types of sugar may do as well, e.g. sugar alcohols (polyalcohol, polyol).

The sugar used in the sugar coating may according to specific embodiments also be an artificial sweetener, being low or substantially free of calories and less caries promoting than regular sugar, or a combination with sugar and/or sugar alcohol.

Film coating involves the deposition, usually by a spray method, of a thin film of polymer surrounding the core. The solution may be sprayed on to a rotated, mixed bed. The drying conditions permit the removal of the solvent so as to leave a thin deposition of coating material around each core.

The composition of the coating solutions and suspensions may differ during different parts of the process.

Press coating involves the compaction of granular material around an already manufactured core. Using press/compression coating, a further core is pressed on the outside of the initial core/cores.

Example 1 - 4 describes four different coatings and coating compositions that may be used according to the invention, *i e* sugar coating in example 1, film coating in example 2, press coating in example 3 and melt coating in example 4, all onto a chewing gum core. The coating is buffered in each case and contains nicotine as well. The coatings in example 1 - 4 may be combined with different cores. Examples of cores are given in Example 5 and further described below.

If nicotine hydrogen tartrate (NHT) is used as the nicotine form then NHT and buffer are suitably separated from each other in the coating by keeping them in separate layers, especially when sugar coating is used. A moisture barrier between the NHT-containing layer and the coating comprising the buffer may be applied to prevent interaction between the acid salt NHT and the buffer during the coating process. Suitable moisture barriers are e.g. ethylcellulose or a combination of ethylcellulose and hydroxypropyl methylcellulose (HPMC) and/or plasticizer from an organic solvent or solvent mixture, aqueous ethylcellulose dispersion such as Aquacoat EDC(FMC Corp.) or Surelease(Colorcon) preferably in combination with plasticizer, Sepifilm LP 007 or LP 010(Seppic) - based mainly on HPMC and stearic acid -, Opadry AMB or High Performance Opadry II(Colorcon) - based mainly on polyvinylalcohol -, and polymethacrylates as Eudragit L30 D-55 or
EPO(Röhm). Depending on the type of barrier film selected the moisture barrier preferably accounts for a weight of around 0.3% to around 5 % of the total weight of the coating.

An additive may be added to the coating or the core/s. Additives are further described in the paragraph *Other additives to the chewing gum.*

### The core

The amount of gum base in the coated chewing gum according to the invention is about 15 - 80 % by weight of the total gum core, and preferably at least about 40 %. The amount of gum base employed for the most desirable slow release of nicotine is usually in the higher ranges when nicotine is employed *per se* or when an absorbed form is used.

The gum base may be of any conventional nature known in the art. For example it may comprise a gum base of natural or synthetic origin readily available from a commercial source. Natural gum bases include, e g chicle, jelutong-, lechi de caspi-, soh-, siak-, katiau-, sorwa-, balata-, pendare-, malaya-, and peach gums, natural cautchouc and natural resins such as dammar and mastix. Synthetic gum bases are a mixture of:
- elastomers (polymers, masticating substances),
- plasticizer (resin, elastomers, solvent, hydrophobic resin),
- filler (texturizer, water-insoluble adjuvant),
- softener (fat),
- emulsifier,
- wax,
- antioxidant,
- and anti-tacking agents (vinyl polymer, hydrophilic resin).

Other examples of gum bases are gums including agar, alginate, arabic gum, carob gum, carrageenan, ghatti gum, guar gum, karaya gum, pectin, tragacanth gum, locust beam gum, gellan gum and xanthan gum.

Examples of gelling agents comprise gum arabic, starch, gelatine, agar, and pectin.

When the nicotine in any form and the buffering agent or agents are incorporated in the chewing gum mass in accordance with the present invention, it is possible to employ a wide variety of chewing gum compositions and amounts of the chewing gum base. Different chewing gum products may be composed depending on the consumers preference and the purpose of use, in respect of the nicotine level, nicotine distribution and other additives.

### The active ingredient

According to the invention, the chewing gum product comprises nicotine in any form and/or a nicotine mimicking agent. In specific embodiments, the nicotine is part of the at least one coating layer or at least one of the at least one coating layers.

In still further embodiments, the nicotine is a part of the chewing gum core or at least one of the chewing gum cores.

In still even further embodiments, the nicotine is part of the at least one coating layer or at least one of the at least one coating layers and the chewing gum core or at least one of the chewing gum cores to give a fast transmucosal uptake of the nicotine in the oral cavity of a subject so as to obtain a rapid kick or reduction of the urge to smoke and/or use tobacco. Thereby may also be achieved a systemic maintenance level of nicotine.

The nicotine should be in a saliva soluble form to facilitate the release of the agent into the saliva in the oral cavity and, further, the subsequent uptake of the nicotine from the saliva in the oral cavity into the systemic circulation of the subject.

In preferred embodiments, the nicotine in any form is selected from the group consisting of a nicotine salt, the free base form of nicotine, a nicotine derivative, such as a nicotine cation exchanger, a nicotine inclusion complex or nicotine in any non-covalent binding; nicotine bound to zeolites; nicotine bound to cellulose or starch microspheres; and mixtures thereof.

Still, further the inclusion complex may be a cyclodextrin, such as β-cyclodextrin.

Even further the cation exchanger may be a polyacrylate.

Even more further, the nicotine salt may be a tartrate, hydrogen tartrate, citrate or malate.

According to the invention, the uptake of the nicotine through any tissue or mucosa in the oral cavity, i e the absorption kinetics, is improved in relation to the uptake obtained by known chewing gum products for nicotine uptake.

The nicotine may act as a stimulant to e g obtain a rapid reduction of the urge to smoke or to use tobacco.

With nicotine it is intended to include nicotine, 3-(1-methyl-2-pyrrolidinyl)-pyridine, with its base form, including synthetic nicotine as well as nicotine extracts from tobacco plants, or parts thereof, such as the genus Nicotiana alone or in combination; or pharmaceutically acceptable salts.

The most preferable embodiment incorporates nicotine as the free base form or as a water-soluble pharmaceutically acceptable salt, either *per se* or adsorbed on a adsorbent, or as a complex with a cation exchanger or mixtures of the foregoing, as an inclusion complex, such as a cyclodextrin complex, e g β-cyclodextrin, but any other suitable pharmaceutically acceptable form may also be employed.

Numerous nicotine salts are known, and may be used, e g the salts presented in Table 1, such as preferably the tartrate, hydrogen tartrate, citrate, malate, and/or hydrochloride.

**Table 1 Possible acids used for nicotine salt formation**

| Acid | Molar ratio* of acid:nicotine |
|---|---|
| Formic | 2:1 |
| Acetic | 3:1 |
| Propionic | 3:1 |
| Butyric | 3:1 |
| 2-Methylbutyric | 3:1 |
| 3-Methylbutyric | 3:1 |
| Valeric | 3:1 |
| Lauric | 3:1 |
| Palmitic | 3:1 |
| Tartaric | 2:1 |
| Citric | 2:1 |
| Malic | 2:1 |
| Oxalic | 2:1 |
| Benzoic | 1:1 |
| Gentisic | 1:1 |
| Gallic | 1:1 |
| Phenylacetic | 3:1 |
| Salicylic | 1:1 |
| Phthalic | 1:1 |
| Picric | 2:1 |
| Sulfosalicylic | 1:1 |
| Tannic | 1:5 |
| Pectic | 1:3 |
| Alginic | 1:2 |
| Hydrochloric | 2:1 |
| Chloroplatinic | 1:1 |
| Silicotungstic | 1:1 |
| Pyruvic | 2:1 |
| Glutamic | 1:1 |
| Aspartic | 1:1 |

| | |
|---|---|
| * recommended upon production | |

The product according to the invention may also comprise a nicotine mimicking agent. Such an agent may be any suitable agent with an acrid burning taste. Examples of nicotine mimicking agents with an acrid burning taste are capsaicin, piperine and zingerone.

One or more additives may be added to the coating or the core/s. Additives are further described in the paragraph *Other additives to the chewing gum.*

### Amount and distribution of the nicotine in the coated chewing gum

The nicotine in any form is according to the invention formulated to provide the subject with a dose to achieve an effect. The effect may be to provide a sense of smoking satisfaction without smoking. Another effect of the administered nicotine in any form may be a reduction of the urge to smoke or use tobacco.

The effect may also be a combination of reduction of said urge and providing a sense of smoking satisfaction without smoking. The amount of the nicotine should be sufficient to provide such an effect in a subject. This amount may, of course, vary from person to person.

According to the invention, embodiments of the chewable gum product comprise embodiments wherein nicotine in any form is present in an amount of 0.05 - 10 mg calculated as the free base form of nicotine per piece coated chewing gum product. This may in different embodiments include 0.05, 0.5, 1, 2, 3, 4, 5, 6, 7, 8, 9, or 10 mg calculated as the free base form of nicotine per piece coated chewing gum product.

Still preferred embodiments may contain embodiments where the nicotine in any form is present in an amount of 0.5 - 6 mg calculated as the free base form of nicotine per piece coated chewing gum product.

Even more preferred embodiments contain the nicotine in any form in an amount of 0.5 - 4 mg calculated as the free base form of nicotine per piece coated chewing gum product.

According to certain embodiements of the invention, the nicotine in any form is part of the at least one coating layer or at least one of the at least one coating layer.

The nicotine in any form may be in an amount of 0 - 10 mg calculated as free base form in at least one of the at least one coating layer. Still further embodiments comprise nicotine in an amount of 0.2 - 6 mg in at least one of the at least one coating layers, or even more preferably, in an amount of 0.5 - 5 mg in at least one of the at least one coating layer.

The nicotine in any form may be distributed in the core and/or different coating layers in different embodiments. Different distributions of the nicotine throughout the coated chewing gum will imply administration of the nicotine to the subject in different ways. This may, then, provide several possibilities to adjust the composition of the coated chewing gum according to different needs of different subjects depending on the urge to smoke or use tobacco of the subject.

### Release and uptake of nicotine

Presently existing nicotine chewing gums provide a slow release and a slow uptake of the nicotine compared to smoking. The slow uptake of the nicotine provides a tₘₐₓ, i e the time-point where the nicotine has its maximum level measured in the plasma of venous blood, after a single dose at about 30 minutes after administration. The tₘₐₓ is initially preceded by a lag period of a couple of minutes where no nicotine can be detected in the blood. Then, the plasma level is gradually increasing till the tₘₐₓ is reached.

The time point for reaching a sense of satisfaction or reduction of urge to smoke or use tobacco after the initial lag period is individual, but may in existing chewing gums generally be reached after approximately 30 minutes when regarded as coinciding with tₘₐₓ. According to the present invention, such a sense of satisfaction may be reached after a shorter period of time due to a rapid initial burst dose of nicotine in the coating followed by a rapid transmucosal uptake in the oral cavity due to the buffered coating.

The release of the nicotine in the coated chewing gum according to the invention proceeds in at least one step.

I) If the nicotine is, as in preferred embodiments, in a defined amount, such as the amounts described above according to different embodiments, in at least one of the at least one coating layers defined above the release of the nicotine takes place when the coating of the coated chewing gum is allowed to melt, disintegrate or dissolve to uncover the chewable gum core in said product. The nicotine and forms thereof is released from the coating into the saliva in the oral cavity during the time period when the coating is allowed to melt, disintegrate or dissolve.

When the at least one coating layer is allowed to melt, disintegrate or dissolute a chewable gum is uncovered. The nicotine in any form may then further be released to the subject.

II) The nicotine in any form from the chewable gum is released by controlled release, e g by chewing the gum core whereby the chewing is controlling the amount of released nicotine from the gum core. The release of the nicotine is thereby sustained over a period of time. This period of time may be, in different embodiments about 10, 20, 30, 40, 50, or 60 minutes.

The dissolution of the one or more buffering agents in the coating, and optionally in the core(s), provides for optimized adjustment of the pH of the liquid in the oral cavity.

The release may be varied by the incorporation of the nicotine in any form in a given quantity into the coating layers and/or the gum core.

Suitable cat ion exchangers are given in table 2 and are further disclosed in US 3 845 217, hereby included as a reference. Preferred are nicotine cation exchangers of polyacrylates, such as the Amberlite collection from Rohm & Haas in table 2.
Examples of different salts are given in Table 1.

**Table 2 Representative cation exchangers**

| Name | Manufacturer |
|---|---|
| Amberlite IRC 50 | Rohm & Haas |
| Amberlite IRP 64 | Rohm & Haas |
| Amberlite IRP 64M | Rohm & Haas |
| BIO-REX 70 | BIO-RAD Lab. |
| Amberlite IR 118 | Rohm & Haas |
| Amberlite IRP 69 | Rohm & Haas |
| Amberlite IRP 69M | Rohm & Haas |
| BIO-REX 40 | BIO-RAD Lab. |
| Amberlite IR 120 | Rohm & Haas |
| Dowex 50 | Dow Chemical |
| Dowex 50W | Dow Chemical |
| Duolite C 25 | Chemical Process Co |
| Lewatit S 100 | Farbenfabriken Bayer |
| Ionac C 240 | Ionac Chem. |
| Wofatit KP S 200 | I.G. Farben Wolfen |
| Amberlyst 15 | Rohm & Haas |
| Duolite C-3 | Chemical Process |
| Duolite C-10 | Chemical Process |
| Lewatit KS | Farbenfabriken Bayer. |
| Zerolit 215 | The Permutit Co. |
| Duolite ES-62 | Chemical Process |
| BIO-REX 63 | BIO-RAD Lab. |
| Duolite ES-63 | Chemical Process |
| Duolite ES-65 | Chemical Process |
| Ohelex 100 | BIO-RAD Lab. |
| Dow Chelating Resin A-1 | Dow Chemical Company |
| Purolite C115HMR | Purolite International Ltd. |
| CM Sephadex C-25 | Pharmacia Fine Chemicals |
| SE Sephadex C-25 | Pharmacia Fine Chemicals |

Not only the amount of the nicotine released from the different parts of the chewing gum product is of value as known in the art, but also, according to the present invention the specific transmucosal uptake from the oral cavity of the nicotine to the systemic circulation of the subject whereby the one or more buffering agents account for provision of a suitable adjustment of the pH of the liquid of the oral cavity. The uptake according to the invention is further discussed in the method paragraphs below.

### Other additives to the chewing gum

Other additives may be added optionally to the chewing gum core and/or to coating layers in the chewing gum.

Optional additives comprise at least one or more additive selected from the group consisting of stabilisers, such as preservatives, e g antioxidants; softeners, thickening agents, emulsifiers, glidants, lubricants, sweeteners, flavours, aromatics, enhancers, colouring agents, vitamins, minerals, fluorine and tooth whitening agents and mixtures thereof. According to the invention, at least one of such additives is optionally added to the product.

Enhancers are added essentially to improve, i e increase, the transmucosal uptake from the oral cavity.

Sweeteners are added essentially to improve the taste.

Sweeteners comprise one or more synthetic or natural sugars, i e any form of carbohydrates suitable for use as sweetener, as well as so called artificial sweeteners such as saccarin, sodium saccarin, aspartame, e g NutraSweet^{®}, acesulfame K or acesulfame, potassium acesulfame, thaumatin, glycyrrhizin, sucralose, dihydrochalcone, alitame, miraculin, monellin, stevside.

Suitable sweeteners may be selected from the group consisting of sugar alcohols, such as sorbitol, xylitol, single sugars including sugars extracted from sugar cane and sugar beet (sucrose), dextrose (also called glucose), fructose (also called leavulose), and lactose (also called milk sugar); sorbitol, mannitol, glycerol, xylitol, maltitol syrup (or hydrogenated starch hydrolyzate), isomalt, lactitol; and mixtures of sugars including glucose syrup, e g starch hydrolysates, containing a mixture of dextrose, maltose and a range of complex sugars, invert sugar syrup, e g sucrose inverted by invertase (also called sucrase or sacchrase) containing a mixture of dextrose and fructose, high sugar content syrups such as treacle and honey containing a mixture of particular leavulose, dextrose, maltose, lactitole, sucrose, resins, dextrin and higher sugars; and malt or malt extracts.

The flavour and aroma additives may comprise one or more synthetic or natural flavouring or aromatizing agents.

Flavour and aroma agents may be selected from essential oils including distillations, solvent extractions, or cold expressions of chopped flowers, leaves, peel or pulped whole fruit comprising mixtures of alcohols, esters, aldehydes and lactones; essences including either diluted solutions of essential oils, or mixtures of synthetic chemicals blended to match the natural flavour of the fruit, e g strawberry, raspberry and black currant; artificial and natural flavours of brews and liquors, e g cognac, whisky, rum, gin, sherry, port, and wine; tobacco, coffee, tea, cocoa, and mint; fruit juices including expelled juice from washed, scrubbed fruits such as lemon, orange, and lime; spear mint, pepper mint, wintergreen, cinnamon, cacoe/cocoa, vanilla, liquorice, menthol, eucalyptus, aniseeds nuts (e g peanuts, coconuts, hazelnuts, chestnuts, walnuts, colanuts), almonds, raisins; and powder, flour, or vegetable material parts including tobacco plant parts, e g genus Nicotiana, in amounts not contributing significantly to the level of nicotine, and ginger.

Colouring additives may be selected from dyes being approved as a food additive.

Stabilizing additives may be selected from the group consisting of antioxidants including vitamin E, i e tocopherole, ascorbic acid, sodium pyrosulfite, butylhydroxytoluene, butylated hydroxyanisole, edetic acid and edetate salts ; and preservatives including citric acid, tartaric acid, lactic acid, malic acid, acetic acid, benzoic acid, and sorbic acid. Preferred embodiments comprise an antioxidant as the stabiliser, and even more preferably the antioxidant vitamin E and/or butylated hydroxytoluene (BHT).

### Method for delivering nicotine in any form to a subject

According to the invention, a method for delivering nicotine in any form to a subject comprises the steps of
a) administering to a subject a coated chewing gum product containing nicotine in any form according to the invention into the oral cavity of the subject, and
b) allowing the nicotine in any form in the coated chewing gum product to be released in the saliva in the oral cavity and absorbed into the blood plasma of the subject.

According to the invention, the transmucosal uptake of the nicotine in the oral cavity is more rapid than with presently known chewing gum not being buffered in the coating. One embodiment results in a tₘₐₓ of nicotine in venous blood of the subject after about 5 - 20 minutes.

In still one embodiment, said nicotine in any form is absorbed resulting in a tₘₐₓ of nicotine in venous blood of the subject after about 3 - 15 minutes.

The method for delivering nicotine in any form may further comprise the steps of
c) administering the nicotine in any form in a sustained way over a period of time to the subject.

Such a time period may be at least 10, 20, 30, 40, 50 or 60 minutes.

### Method for obtaining reduction of the urge to smoke or use of tobacco

A method for obtaining reduction of the urge to smoke or use tobacco containing material and/or for providing a sense of smoking satisfaction without smoking according to the invention comprises the steps of
a) replacing at least partly the tobacco containing material with a coated chewing gum according to any of claims 1 - 20,
b) administering to a subject a coated chewing gum product containing nicotine in any form according to any of claims 1 - 20 into the oral cavity of the subject, and
c) allowing the nicotine in any form in the coating of the coated chewing gum product to be released in the saliva in the oral cavity and absorbed by the subject.

In one embodiment said nicotine in any form results in a tₘₐₓ of nicotine in venous blood of the subject after about 5 - 20 minutes.

In a further embodiment, the absorbing of said nicotine released from the at least one coating layer is resulting in a tₘₐₓ of nicotine in venous blood of the subject after about 3 - 15 minutes.

In still another embodiment, the method according to the invention further comprises the steps of administering the nicotine in any form in a sustained way over a period of time to the subject.

The period of time may be at least 10, 20, 30, 40, 50 or 60 minutes.

Even further embodiments of the method for delivering nicotine to a subject may comprise the steps of combining at least one other method for obtaining reduction of the urge to smoke or use of tobacco.

Tobacco containing material may be material used for e g smoking, snuffing or chewing and may comprise a cigarette, a cigarr, snuff, pipe tobacco and also chewing tobacco.

The coated chewing gum product may be used for obtaining a quick and/or sustained and/or complete reduction of the urge to smoke or use of tobacco and/or for providing a sense of smoking satisfaction without smoking as further discussed below.

The fast relief provides the subject with a sense of rapid smoking satisfaction without smoking. Such a satisfaction will decrease the craving more rapidly than other known nicotine chewing gum products.

The quick craving relief is obtained when a dosage of nicotine is released from at least one of the at least one coating layers of the coated chewing gum in embodiments wherein nicotine is in the coating layers in the presence of one or more buffering agents in the coating and optionally in the core(s). This provides the subject with an initial rapid transmucosal uptake of nicotine in the oral cavity that will induce an initial peak, a maximum, in the blood plasma nicotine levels after a certain time, tₘₐₓ. The result will be that the subject gets a feeling or sense of satisfaction and the initial craving will disappear.

One embodiment reduces the urge to smoke or use of tobacco by reaching a tₘₐₓ of nicotine in venous blood of the subject after about 5 - 20 minutes by the use of a coated chewing gum according to the invention.

Still one embodiment reduces the urge to smoke or use of tobacco quickly resulting in a tₘₐₓ of nicotine in venous blood of the subject after about 3 - 15 minutes when said nicotine in any form is released from the at least one coating layer after use of a coated chewing gum according to the invention.

### Sustained reduction of the urge to smoke or use of tobacco

Still, to continue the feeling or sense of satisfaction of the subject, and to avoid that the craving returns, a sustained craving relief may be obtained after the initial craving relief. A sustained craving relief is obtained by chewing the gum part, or core, of the coated chewing gum to allow a sustained uptake of the nicotine. The sustained craving relief and/or feeling or sense of satisfaction of the subject will continue as long as the subject maintains the blood plasma levels of nicotine at a level high enough to reach this sense of feeling.

The subject may achieve this by chewing the gum part of the chewing gum over a period of time, such as 10, 20, 30, 40, 50, or even 60 minutes or longer, e g a slow release of the nicotine caused by a controlled release, e g by individual chewing.

### Cessation of the urge to smoke or use of tobacco

For some of the users, it may be a goal to terminate the usage of nicotine completely, due to several reasons e g health, economical, social or behavioural. This may be achieved by further decreasing the amount of nicotine in any form gradually over time. In a specific embodiment of the invention, the method described above for obtaining craving relief may further comprise the steps of decreasing the amount of nicotine in the total coated chewing gum product described above gradually over time, so as to achieve a complete relief of tobacco craving. This method results in a weaning process gradually over time.

Different types of smokers reach the sense of reduced craving at different plasma levels of nicotine. This may, of course, affect the individual types of administration programs of a coated chewing gum according to the invention. Different types of smokers include e g peak seekers or smokers that crave for a plasma level of nicotine constantly being above the level for withdrawal symptoms.

One strategy may be to lower the frequency of the administered coated chewing gums. Other embodiments include varying the dose of the nicotine in said gums as well as the combination of these two. Also, the strategy may include a coated chewing gum with substantially no nicotine in any form. Such a gum may be administered at the end of the treatment period, when the craving is low or substantially absent.

### Systems for delivering nicotine and for obtaining craving relief

According to the invention there is a system for delivering nicotine in any form to a subject. Such a system comprises a coated chewing gum according to the invention and at least one other means for obtaining reduction of the urge to smoke.

Another system according to the invention may also be a system for obtaining reduction of the urge to smoke or use of tobacco and/or for providing a sense of smoking satisfaction without smoking. Such a system comprises a coated chewing gum product according to the invention and at least one other method for obtaining reduction of the urge to smoke or use tobacco. Other methods may also be a concomitant or concurrent method selected from the group consisting of administration through mouth sprays, nasal sprays, transdermal patches, inhaling devices, lozenges, tablets and parenteral methods, subcutaneous methods, intravenous methods, rectal methods, vaginal methods and transmucosal methods; or use of tobacco.

In a specific embodiment, the at least other method comprises administration of nicotine.

### Use of the coated chewing gum

The use of the coated chewing gum product according to the invention is for obtaining a fast and/or sustained and/or complete reduction of the urge to smoke and use tobacco or for providing a sense of smoking without smoking as described above.

The dose of the nicotine is chosen to give the subject an individual sensory perception and satisfaction with an effect of the nicotine in any form. The use of the coated chewing gum product may also be a sole use according to the invention or a combination with other means or methods known in the field of drug abuse. Specifically, the present invention may be used in combination with other means as described above in the methods in the paragraphs above.

The use may give a quick reduction of the urge to smoke or use tobacco obtained reaching a tₘₐₓ of nicotine in venous blood after e g about 5 - 20 minutes.

In a specific embodiment, the use of the coated chewing gum according to the invention will reduce the urge to smoke or use tobacco by reaching a tₘₐₓ of nicotine in venous blood of the subject after about 3 - 15 minutes when said nicotine in any form is released from the at least one coating layer.

According to the invention, a use of a coated chewing gum product according to the invention is also disclosed for delivering nicotine in any form to a subject.

In one embodiment, the delivering of nicotine in any form results in a tₘₐₓ of nicotine in venous blood of the subject after about 5-20 minutes.

In still another embodiment, the delivering of nicotine in any form results in a tₘₐₓ of nicotine in venous blood of the subject after about 3-15 minutes when said nicotine in any form released from the at least one coating layer is absorbed.

### Production of the coated chewing gum

Coated chewing gum products according to the invention can be maintained in several production steps depending on the total number of cores and the total number of coated layers to be included.

A method for the production of the coated chewing gum according to the invention is disclosed.

The method comprises the steps of
a) providing at least one chewing gum core, and/or providing at least one nicotine containing chewing gum core,
b) providing nicotine in any form,
c) providing at least one coating layer that is buffered,
d) adding the nicotine in any form to the at least one chewing gum core and/or to the at least one coating, and
e) coating the at least one chewing gum core with the at least one coating layer that is buffered.

The method may in specific embodiments further comprise
f) buffering the at least one chewing gum core, and/or
g) providing at least one coating layer not being buffered, and optionally
h) adding the nicotine in any form to at least one of said at least one coating layer not being buffered, and optionally
i) providing the nicotine in the coating and the buffer in the coating in separate layers, preferably separated by a moisture barrier.

In one embodiment, the nicotine is selected from the group consisting of a nicotine salt, the free base form of nicotine, a nicotine derivative, such as a nicotine cation exchanger, a nicotine inclusion complex or nicotine in any non-covalent binding; nicotine bound to zeolites; nicotine bound to cellulose or starch microspheres; and mixtures thereof.

The at least one coating layer may in some embodiments be buffered by the use a buffer selected from the group consisting of a carbonate buffer, such as the carbonate, bicarbonate, sesquicarbonate of an alkali metal, e g potassium, sodium; or ammonium; sodium glycinate, alkali metal phosphate, sodium or potassium glycerophosphate, trisodium or tripotassium citrate, and mixtures thereof wherein the at least one coating layer is buffered in such a way that upon administration of the gum the pH of the saliva is increased by 0.3 - 4 pH units. The buffering may be transient.

In still further embodiments, the at least one coating layer is buffered in such way that upon administration of the gum the pH of the saliva is increased by 0.5 - 2 pH units. The core/pellet composition may be formed simply by mixing, rolling and scoring or compression of the chewing gum base with at least one of the forms of nicotine, e g the nicotine-ion exchanger complex, or the nicotine as a free base or a salt. Before adding any solid component, except for the gum base, it is desirable to grind and size the solid component first, to ensure good distribution. The mixing is preferably conducted at a suitably elevated temperature depending on the viscosity of the gum core used. The increase in temperature decreases the viscosity of the gum and thereby enables the nicotine and other additives to be evenly and intimately distributed within the core/pellet of the chewing gum.

Example 5 describes the mixing, rolling and scoring as well as the compression of gum cores. In principle, this procedure may be applied for coated chewing gum products up to at least 10 mg unit formula.

Mixing, rolling and scoring is done by a conventional procedure. Double sigma blade mixers are used for mixing the gum base with the other components of the formulation. The gum base is softened in the mixer. By heat (from the heating jacket) and mixing, the gum base becomes plastic. So, the softened base is mixed with the liquid components, e g flavours, liquid, sorbitol and glycerol, nicotine in base form, when used, and the solid materials, e g nicotine in any form other than in liquid form, buffer, bulk sweetener, colour as a powder mixture. The warm mass is discharged from the mixer in form of loaves stacked on trays on a truck and stored in a conditioned area until the next step starts. This is to cool the gum.

After this, the rolling and scoring takes place. The gum is extruded into a thick sheet, which is rolled by multiple sets of calender rolls to the correct thickness. The scoring rolls, usually two sets, cut the gum into the correct size.

The sheets are then transferred to a conditioned area on trays, where the sheets are cooled to make them brittle enough to be broken. The conditioned gum sheets are then passed through a breaker, which is a rotating drum that parts the sheets into separate pieces of gum along the scores.

At a sorting stage deformed gums are sorted away. The accepted gums are passed through a metal detector.

Chewing gums produced by compression, i e tabletted gums are made out of a special gum base. Such a gum base gives a rapid hydration of the cud in the mouth. High velocity mixers can be used for granulation to give correctly sized particles of the mixture. This mixture is then compressed in a tablet machine.

At a sorting stage deformed gums are sorted away. The accepted gums are passed through a metal detector.

In one embodiment of the method disclosed, the provision of the at least one chewing gum core in step a) above comprises the further steps of
a1) providing a gum core dough,
a2) mixing, rolling and scoring; molding; or extruding the gum dough.

In still another embodiment, the provision of the chewing gum core in step a) is obtained by direct compressing of the ingredients.

Conveniently, the compositions of additives according to the invention, e g the buffer system, are made simultaneously, according to known procedures in the art for formulating the buffers. Depending on the physical properties of the buffer system incorporated, it may be convenient to add the buffer system/s either with the liquid part or with he solid part of the composition. In the case of buffering systems available as fine powders, it may, of course, be most convenient to add those powders with the solid, powdered part of other additives.

The gum mass with additives is cooled, rolled, scored and hardened sufficiently, and then coated according to the paragraph *The coating of the gum cone*/*pellet.*

According to the method disclosed in the invention, some embodiments are disclosed where the coating of the at least one chewing gum core with at least one layer of the at least one buffered coating comprises the steps of
a) film coating, and/or
b) press coating, and/or
c) sugar coating, and/or
d) melt coating.

The product may then be analysed and further wrapped.

### Use fon therapy and treatment

The coated chewing gum product according to the invention may be used in therapy. Said therapy may be a treatment of a disease selected from the group consisting of Alzheimer's disease, Crohn's disease, Parkinson's disease, Tourette's syndrome, ulcerous colitis and post-smoking-cessation weight control.

The nicotine may also be used for the production of a chewing gum product according to the invention for the treatment of a disease selected from the group consisting of Alzheimer's disease, Crohn's disease, Parkinson's disease, Tourette's syndrome, ulcerous colitis and post-smoking-cessation weight control.

Also disclosed is the use of a chewing gum product for the production of a nicotine containing chewing gum product according to the invention for the treatment of a disease selected from the group consisting of Alzheimer's disease, Crohn's disease, Parkinson's disease, Tourette's syndrome, and ulcerous colitis.

### Analysis of nicotine

The analysis of nicotine uptake and effect according to the invention may be done according to standard procedure known in the art, e g using a bioanalys for the determination of nicotine or its metabolites in the plasma of a subject.

### Examples

The below examples are illustrative and non-limiting.

### Example 1 Buffered sugar coating

This example describes without limiting the invention a sugar coating, wherein the coating is buffered and wherein the nicotine is in the coating.

### Objective

The objective of this example is to provide a sugar coated chewing gum, wherein the coating is buffered and wherein the nicotine is in the coating. The nicotine is in the amount of 1, 2, 4 or 5 mg, respectively.

### Material sugar coating*

### A. Nicotine free base

| | 1 mg | 2 mg | 4 mg | 5 mg |
|---|---|---|---|---|
| | unit formula | unit formula | unit formula | unit formula |
| Component | (mg) | (mg) | (mg) | (mg) |
| Sorbitol | 19,0 | 22,2 | 28,7 | 31,9 |
| Mannitol | 29,4 | 29,4 | 29,4 | 29,4 |
| Xylitol | 162 | 162 | 162 | 162 |
| Water | q.s.^{**} | q.s. | q.s. | q.s. |
| Gelatin | 3,4 | 3,4 | 3,4 | 3,4 |
| Titanium dioxide | 2,5 | 2,5 | 2,5 | 2,5 |
| Sodium carbonate | 10 | 15 | 20 | 20 |
| Nicotine free base | 1 | 2 | 4 | 5 |

| | | | | |
|---|---|---|---|---|
| sugar coating in this example denotes sugar alcohols, not saccharose based sugar. ** q.s. = quantum satis. | | | | |

### B. Nicotine hydrogen tartrate

| | 1 mg | 5 mg |
|---|---|---|
| | unit formula | unit formula |
| Component | (mg) | (mg) |
| Sorbitol | 19,0 | 31,9 |
| Mannitol | 29,4 | 29,4 |
| Xylitol | 162 | 162 |
| Water | q.s. | q.s. |
| Gelatin | 3,4 | 3,4 |
| Sodium carbonate | 15 | 40 |
| Titanium dioxide | 2,5 | 2,5 |
| Nicotine hydrogen tartrate, N.H.T. | 3,1 | 15,4 |
| (corresponding to nicotine free base) | 1 | 5 |

### Results and discussion

Other amounts of nicotine are possible.

### Example 2 Buffered film coating

This example describes without limiting the invention a film coated chewing gum, wherein the coating is buffered and wherein the nicotine is in the coating.

### Objective

The objective of this example is to provide a film coated chewing gum, wherein the coating is buffered and wherein the nicotine is in the coating. The nicotine is in the amount of 1, 2, 4 or 5 mg, respectively.

### Material film coating

### A. NHT

| Component | 1 mg unit formula (mg) | 2 mg unit formula (mg) | | 4 mg tablet unit formula (mg) | |
|---|---|---|---|---|---|
| HPMC^{a},5cPs | 15,9 | 15,9 | 15,9 | 20 | 20 |
| PEG^{b}4000/6000 | 4,8 | 0 | 4,8 | 0 | 4,8 |
| Water | 27 | 27 | 27 | 34 | 34 |
| Ethanol | 221 | 221 | 221 | 278 | 278 |
| NHT, | 3,1 (1) | 6,2 (2) | 6,2 (2) | 12,3 (4) | 12,3 (4) |
| (corresponding to nicotine free base, mg) | | | | | |
| Paraffin wax | 0,7 | 0,7 | 0,7 | 0,7 | 0,7 |
| Sodium carbonate | 15 | 20 | 20 | 35 | 35 |

| | | | | | |
|---|---|---|---|---|---|
| ^{a}= HPMC=hydroxypropyl methylcellulose ^{b} = PEG=polyethylene glycol | | | | | |

### B. Nicotine free base

| | 1 mg | 2 mg | 4 mg | 5 mg |
|---|---|---|---|---|
| | unit formula | unit formula | unit formula | unit formula |
| Component | (mg) | (mg) | (mg) | (mg) |
| HPMC^{a},5cPs | 19,0 | 19,0 | 19,0 | 19,0 |
| PEG^{b}4000/6000 | 4,8 | 4,8 | 4,8 | 4,8 |
| Water | 27 | 27 | 27 | 27 |
| Ethanol | 221 | 221 | 221 | 221 |
| Paraffin wax | 0,7 | 0,7 | 0,7 | 0,7 |
| Sodium carbonate | 15 | 15 | 20 | 20 |
| Nicotine free base | 1 | 2 | 4 | 5 |

### Example 3 Buffered press coating

This example describes without limiting the invention a press coated chewing gum, wherein the coating is buffered and wherein the nicotine is in the coating.

### Objective

The objective of this example is to provide a press coated chewing gum, wherein the coating is buffered and wherein the nicotine is in the coating. The nicotine is in the amount of 1, 2, 4 or 5 mg, respectively.

### Material press coating

### A. NHT

| Component | 1 mg unit formula | 2 mg unit formula | 4 mg unit formula | 5 mg unit formula |
|---|---|---|---|---|
| Xylitab 100 | 734 | 731 | 725 | 722 |
| HPMC,3cPs | 238 | 238 | 238 | 238 |
| Sodium carbonate | 10 | 20 | 30 | 40 |
| Magnesium stearate | 10 | 10 | 10 | 10 |
| Nicotine hydrogen | 3,1 | 6,2 | 12,3 | 15,4 |
| tartrate, corresponding to nicotine free base | 1 | 2 | 4 | 4 |

### B. NRC or NCC

| | 2 mg | | 4 mg | |
|---|---|---|---|---|
| | unit formula (mg) | | unit formula (mg) | |
| Component | NRC | NCC | NRC | NCC |
| Xylitab 100^{a} | 727 | 720 | 717 | 703 |
| HPMC,3cPs | 238 | 238 | 238 | 238 |
| Sodium carbonate | 15 | 15 | 30 | 30 |
| Magnesium stearate | 10 | 10 | 10 | 10 |
| NCR | 10 | - | 20 | - |
| (corresponding to nicotine free base) | 2 | - | 4 | - |
| NCC | - | 17,1 | - | 34,2 |
| (corresponding to nicotine free base) | - | 2 | - | 4 |

### Example 4 Buffered melt coating

This example describes without limiting the invention a melt coated chewing gum, wherein the coating is buffered and wherein the nicotine is in the coating.

### Objective

The objective of this example is to provide a melt coated chewing gum, wherein the coating is buffered and wherein the nicotine is in the coating. The nicotine is in the amount of 1, 2, 4 or 5 mg, respectively.

### Material melt coating

### A. NHT

| Component | 1 mg unit formula | 2 mg unit formula | 4 mg unit formula | 5 mg unit formula |
|---|---|---|---|---|
| Hydrogenated vegetable oil,type II, (Akopol E) | 176 | 176 | 176 | 176 |
| Cocoa, alkalized | 198 | 197 | 192 | 191 |
| Aspartame | 2,4 | 2,4 | 2,4 | 2,4 |
| Sodium carbonate | 15 | 20 | 30 | 40 |
| Lecithin | 4 | 4 | 4 | 4 |
| Nicotine hydrogen | 3,1 | 6,2 | 12,3 | 15,4 |
| tartrate, (corresponding to nicotine base, mg) | 1 | 2 | 4 | 5 |

### B. Nicotine free base

| Component | 1 mg unit formula | 2 mg unit formula | 4 mg unit formula | 5 mg unit formula |
|---|---|---|---|---|
| Hydrogenated vegetable oil,type II, (Akopol E) | 176 | 176 | 176 | 176 |
| Cocoa,alkalized | 198 | 197 | 192 | 191 |
| Aspartame | 2,4 | 2,4 | 2,4 | 2,4 |
| Sodium carbonate | 15 | 15 | 20 | 20 |
| Lecithin | 4 | 4 | 4 | 4 |
| Nicotine free base | 1 | 2 | 4 | 5 |

### Example 5 Manufacture of cores

This example describes without limiting the invention the manufacture of different cores according to the invention.

### Objective

The objective of this example is to provide a core suitable for a chewing gum product according to the invention. The nicotine is incorporated as the free base (NFB), nicotine β-cyclodextrin complex (NCC), nicotine hydrogen tartrate (NHT) or as a nicotine resin complex (NRC). The amount of nicotine in each fomula unit, i e per tablett core, is 1, 2, 4, or 6 mg.

### Principle

The core is formed by a mixing, rolling and scoring process or by a compression process.

### Composition of the cores

### A. Manufactured by tablet compression process.

| | 6 mg | 4 mg | 2 mg | 1 mg |
|---|---|---|---|---|
| | Unit formula | Unit formula | Unit formula | Unit formula |
| **Active ingredients** | (mg) | (mg) | (mg) | (mg) |
| Nicotine resin complex 20% | 30 | 20 | 10 | 6 |
| | | | | |
| **Other ingredients** | | | | |
| Chewing gum base for compression^{a} | 500 | 500 | 500 | 500 |
| Xylitol | 191 | 211 | 221 | 226 |
| Sorbitol | 100 | 100 | 100 | 100 |
| Encapsulated peppermint oil | 100 | 100 | 100 | 100 |
| Sodium carbonate anhydr. | 40 | 30 | 20 | 10 |
| Sodium bicarbonate | - | - | 10 | 20 |
| Magnesium stearate | 15 | 15 | 15 | 15 |
| Talcum | 15 | 15 | 15 | 15 |
| Magnesium oxide | 5 | 5 | 5 | 5 |
| Acesulfame K | 2 | 2 | 2 | 2 |
| Aspartame | 2 | 2 | 2 | 2 |

| | | | | |
|---|---|---|---|---|
| ^{a} Cafosa Gum S/A | | | | |

### B. Manufactured by mixing rolling and scoring

| | 6 mg | 4 mg | 2 mg | 1 mg |
|---|---|---|---|---|
| | Unit formula | Unit formula | Unit formula | Unit formula |
| **Active ingredients** | (mg) | (mg) | (mg) | (mg) |
| Nicotine β-cyclodextrin complex 11,5% | 52,2 | 34,8 | 17,4 | 8,7 |
| **Other ingredients** | | | | |
| Chewing gum base^{a} | 650 | 650 | 650 | 650 |
| Xylitol | 233 | 250 | 268 | 276 |
| Peppermint oil | 30 | 30 | 30 | 30 |
| Sodium carbonate anhydr. | 30 | 25 | 20 | 20 |
| Sodium bicarbonate | - | 5 | 10 | 10 |
| Acesulfame K | 2 | 2 | 2 | 2 |
| Levomenthol | 2 | 2 | 2 | 2 |
| Magnesium oxide | 1 | 1 | 1 | 1 |

| | | | | |
|---|---|---|---|---|
| ^{a} Cafosa Gum S/A | | | | |

### C. Manufactured by mixing rolling and scoring

| | 6 mg | 4 mg | 2 mg | 1 mg |
|---|---|---|---|---|
| | Unit formula | Unit formula | Unit formula | Unit formula |
| **Active ingredients** | (mg) | (mg) | (mg) | (mg) |
| Nicotine free base | 6 | 4 | 2 | 1 |
| | | | | |
| **Other ingredients** | | | | |
| Chewing gum base^{a} | 620 | 620 | 620 | 620 |
| Xylitol | 308 | 310 | 312 | 313 |
| Peppermint oil | 30 | 30 | 30 | 30 |
| Sodium carbonate anhydr. | 30 | 25 | 20 | 10 |
| Sodium bicarbonate | - | 5 | 10 | 20 |
| Acesulfame K | 2 | 2 | 2 | 2 |
| Levomenthol | 2 | 2 | 2 | 2 |
| Magnesium oxide | 2 | 2 | 2 | 2 |

| | | | | |
|---|---|---|---|---|
| ^{a} Cafosa Gum S/A | | | | |

### D. Manufactured by mixing rolling and scoring

| | 6 mg | 4 mg | 2 mg | 1 mg |
|---|---|---|---|---|
| | Unit formula | Unit formula | Unit formula | Unit formula |
| **Active ingredients** | (mg) | (mg) | (mg) | (mg) |
| Nicotine hydrogen | 18,5 | 12,3 | 6,2 | 3,1 |
| tartrate | | | | |
| | | | | |
| **Other ingredients** | | | | |
| Chewing gum base^{a} | 660 | 660 | 660 | 660 |
| Xylitol | 246 | 263 | 269 | 272 |
| Fruit flavour | 30 | 30 | 30 | 30 |
| Sodium carbonate anhydr. | 40 | 30 | 20 | 10 |
| Sodium bicarbonate | - | - | 10 | 20 |
| Acesulfame K | 2 | 2 | 2 | 2 |
| Aspartame | 2 | 2 | 2 | 2 |
| Magnesium oxide | 1 | 1 | 1 | 1 |

| | | | | |
|---|---|---|---|---|
| ^{a} Cafosa Gum S/A | | | | |

### E. Manufactured by mixing rolling and scoring

| | 6 mg | 4 mg | 2 mg | 1 mg |
|---|---|---|---|---|
| | Unit formula | Unit formula | Unit formula | Unit formula |
| **Active ingredients** | (mg) | (mg) | (mg) | (mg) |
| Nicotine resin complex | 30 | 20 | 10 | 5 |
| 20% | | | | |
| | | | | |
| **Other ingredients** | | | | |
| Chewing gum base^{a} | 660 | 660 | 660 | 660 |
| Xylitol | 235 | 255 | 265 | 270 |
| Peppermint oil | 30 | 30 | 30 | 30 |
| Sodium carbonate anhydr. | 40 | 30 | 20 | 10 |
| Sodium bicarbonate | - | - | 10 | 20 |
| Acesulfame K | 2 | 2 | 2 | 2 |
| Levomenthol | 2 | 2 | 2 | 2 |
| Magnesium oxide | 1 | 1 | 1 | 1 |

| | | | | |
|---|---|---|---|---|
| ^{a} Cafosa Gum S/A | | | | |

### Procedures

### I) Mixing, rolling and scoring

Mixing, rolling and scoring is done by a conventional procedure. Double sigma blade mixers are used for mixing the gum base with the other components of the formulation. The gum base is softened in the mixer. By heat (from the heating jacket) and mixing, the gum base becomes plastic. So, the softened base is mixed with the liquid components, e g flavours, liquid, sorbitol and glycerol, when used and the solid materials, e g nicotine in any form, buffer, bulk sweetener, colour) as a powder mixture. The warm mass is discharged from the mixer in form of loaves stacked on trays on a truck and stored in a conditioned area until the next step starts. This is to cool the gum.

After this, the rolling and scoring takes place. The gum is extruded into a thick sheet, which is rolled by multiple sets of calender rolls to the correct thickness. The scoring rolls, usually two sets, cut into the correct size.

The sheets are then transferred to a conditioned area on trays, where the sheets are cooled to make them brittle enough to be broken. The conditioned gum sheets are then passed through a breaker, which is a rotating drum that parts the sheets into separate pieces of gum along the scores.

At a sorting stage deformed gums are sorted away. The accepted gums are passed through a metal detector.

### II) Compression

Chewing gums produced by compression (usually being a dry method), i e tabletted gums are made out of a special gum base. High velocity mixers can be used for granulation to give correctly sized particles of the mixture. This mixture is then compressed in a tablet machine.

At a sorting stage deformed gums are sorted away. The accepted gums are passed through a metal detector.

## Claims

1. A coated chewing gum product comprising at least one buffered or non-buffered chewing gum core, nicotine in any form and/or a nicotine mimicking agent, at least one coating layer and optionally one or more other additive(s), wherein said at least one coating layer is buffered.

2. The product according to claim 1, wherein the at least one core is buffered.

3. The product according to any of claims 1 - 2, wherein the nicotine in any form is a part of the at least one coating layer or at least one of the at least one coating layers.

4. The product according to any of claims 1 - 3, wherein the nicotine in any form is a part of the chewing gum core or at least one of the chewing gum cores.

5. The product according to any of claims 1 - 4, wherein the at least one coating layer is buffered in such a way that upon administration of the gum to a subject the pH of the saliva of the subject is increased by 0.3 - 4 pH units.

6. The product according to claim 5, wherein the at least one coating layer is buffered in such a way that upon administration of the gum to a subject the pH of the saliva of the subject is increased by 0.5 - 2 pH units.

7. The product according to any of claims 1 - 6, wherein the at least one coating layer is buffered by the use of a buffer selected from the group consisting of a carbonate, such as monocarbonate, bicarbonate or sesquicarbonate, glycinate, phosphate, glycerophosphate, acetate, gluconate or citrate of an alkali metal, such as potassium or sodium, or ammonium, and mixtures thereof, whereby optionally the buffer is microencapsulated, and wherein the optional buffering of the at least one chewing gum core is obtained by the use of a buffer according to the above selection.

8. The product according to any of claims 1 - 7, wherein the nicotine in any form is selected from the group consisting of a nicotine salt, the free base form of nicotine, a nicotine derivative, such as a nicotine cation exchanger, a nicotine inclusion complex or nicotine in any non-covalent binding; nicotine bound to zeolites; nicotine bound to cellulose or starch microspheres; and mixtures thereof.

9. The product according to claim 8, wherein the nicotine inclusion complex is a cyclodextrin complex, such as β-cyclodextrin.

10. The product according to claim 8, wherein the nicotine cation exchanger is a polyacrylate cation exchanger.

11. The product according to claim 8, wherein the nicotine salt is a salt formed with tartrate, citrate or malate.

12. The product according to any of claims 1 - 11, wherein the nicotine in any form is present in an amount of 0.05 - 10 mg calculated as the free base form of nicotine per piece coated chewing gum product.

13. The product according to claim 12, wherein the nicotine in any form is present in an amount of 0.5 - 6 mg calculated as the free base form of nicotine per piece coated chewing gum product.

14. The product according to claim 13, wherein the nicotine in any form is present in an amount of 2 - 5 mg calculated as the free base form of nicotine per piece coated chewing gum product.

15. The product according to any of claims 1 - 14, wherein the nicotine in any form is in an amount of 0 - 10 mg calculated as the free base form of nicotine in at least one of the at least one coating layers.

16. The product according to claim 15, wherein the nicotine in any form is in an amount of 0.2 - 6 mg calculated as the free base form of nicotine in at least one of the at least one coating layers.

17. The product according to claim 16, wherein the nicotine in any form is in an amount of 0.5 - 5 mg calculated as the free base form of nicotine in at least one of the at least one coating layers.

18. The product according to any of claims 1 - 17, wherein the nicotine mimicking agent is any agent with acrid burning taste.

19. The product according to claim 18, wherein the nicotine mimicking agent is chosen from any of capsaicin, piperine and zingerone or any mixture thereof.

20. The product according to any of claims 1 - 19, wherein the optional at least one or more additive is selected from the group consisting of stabilisers, such as preservatives, e g antioxidants; softeners, thickening agents, emulsifiers, glidants, lubricants, sweeteners, flavours, aromatics, enhancers, colouring agents, vitamins, minerals, and mixtures thereof.

21. The product according to any of claims 1 - 20, wherein in the coating, especially when sugar coating is used, the nicotine, preferably in the form of nicotine hydrogen tartrate (NHT), and the buffer are separated from each other by being kept in separate layers, whereby said layers optionally are separated by a moisture barrier, said moisture barrier comprising substances chosen from ethylcellulose, hydroxypropyl methylcellulose and polymethacrylates or combinations thereof, preferably combined with one or more plasticizers and/or hydrophobic lipid-based films, such as films comprising stearic acid.

22. A system for delivering nicotine in any form to a subject, comprising a coated chewing gum product according to any of claims 1 - 21 and at least one other means or method for obtaining reduction of the urge to smoke or use of tobacco.

23. A system for obtaining reduction of the urge to smoke or use of tobacco and/or for providing a sense of smoking satisfaction without smoking, comprising a coated chewing gum product according to claim 1 - 21 and at least one other means or method for obtaining reduction of the urge to smoke or use of tobacco.

24. The system according to claim 22 or 23, wherein the at least one other means or method is a concomitant or concurrent means or method selected from the group consisting of administration through mouth sprays, nasal sprays, transdermal patches, inhaling devices, lozenges, tablets and parenteral methods, subcutaneous methods, intravenous methods, rectal methods, vaginal methods and transmucosal methods; or use of tobacco.

25. The system according to claim 24, wherein the at least other means or method comprises administration of nicotine.

26. A method for producing a coated chewing gum product according to claim 1-21, comprising the steps of
a) providing at least one chewing gum core, and/or providing at least one nicotine containing chewing gum core,
b) providing nicotine in any form,
c) providing at least one coating that is buffered,
d) adding the nicotine in any form to the at least one chewing gum core and/or to the at least one coating, and
e) coating the at least one chewing gum core with at least one layer of the at least one buffered coating layer.

27. The method according to claim 26, further comprising the steps of
f) buffering the at least one chewing gum core, and/or
g) providing at least one coating layer not being buffered, and optionally
h) adding the nicotine in any form to at least one of said at least one coating layer not being buffered, and optionally
i) providing the nicotine in the coating and the buffer in the coating in separate layers, preferably separated by a moisture barrier.

28. The method according to any of claims 26 - 27, wherein the nicotine in any form is selected from the group consisting of a nicotine salt, the free base form of nicotine, a nicotine derivative, such as a nicotine cation exchanger, a nicotine inclusion complex or nicotine in any non-covalent binding; nicotine bound to zeolites; nicotine bound to cellulose or starch microspheres; and mixtures thereof.

29. The method according to any of claims 26 - 28, wherein the at least one coating layer is buffered by the use of a buffer selected from the group consisting of a carbonate including bicarbonate or sesquicarbonate, glycinate, phosphate, glycerophosphate or citrate of an alkali metal, such as potassium or sodium, or ammonium, and mixtures thereof, wherein the at least one coating layer is buffered in such a way that upon administration of the gum to a subject the pH of the saliva of the subject is increased by 0.3 - 4 pH units.

30. The method according to claim 29, wherein the at least one coating layer is buffered in such a way that upon administration of the gum to a subject the pH of the saliva of the subject is increased by 0.5 - 2 pH units.

31. The method according to any of claims 26 - 30, wherein the provision of the at least one chewing gum core in step a) comprises the steps of
a1) providing a gum core dough,
a2) mixing, rolling and scoring; moulding; or extruding the gum dough.

32. The method according to any of claims 26 - 30, wherein the provision of the chewing gum core in step a) is obtained by direct compressing of the ingredients.

33. The method according to any of claims 26 - 32, wherein coating the at least one chewing gum core with at least one layer of the at least one buffered coating comprises the steps of
a) film coating, and/or
b) press coating, and/or
c) sugar coating, and/or
d) melt coating.

34. A chewing gum product according to any of claims 1 - 21 for use in therapy.

35. The product according to claim 34, wherein the therapy is treatment of a disease selected from the group consisting of Alzheimer's disease, Crohn's disease, Parkinson's disease, Tourette's syndrome, ulcerous colitis and post-smoking-cessation weight control.

36. Use of nicotine for the production of a chewing gum product according to any of claims 1 - 21 for the treatment of a disease selected from the group consisting of Alzheimer's disease, Crohn's disease, Parkinson's disease, Tourette's syndrome, ulcerous colitis and post-smoking-cessation weight control.

37. Use of a chewing gum for the production of a nicotine containing chewing gum product according to any of claims 1 - 21 for the treatment of a disease selected from the group consisting of Alzheimer's disease, Crohn's disease, Parkinson's disease, Tourette's syndrome, ulcerous colitis and post-smoking-cessation weight control.

## Patentansprüche

1. Überzogenes Kaugummierzeugnis umfassend mindestens einen gepufferten oder nicht gepufferten Kaugummikern, Nikotin in irgendeiner Form und/oder ein Nikotin nachahmendes Agens, mindestens eine Überzugsschicht und optional ein oder mehrere Additiv(e), wobei die genannte mindestens eine Überzugsschicht gepuffert ist.

2. Erzeugnis nach Anspruch 1, wobei der mindestens eine Kern gepuffert ist.

3. Erzeugnis nach irgendeinem der Ansprüche 1 - 2, wobei das Nikotin in irgendeiner Form ein Teil von der mindestens einen Überzugsschicht oder von mindestens einer der mindestens einen Überzugsschicht(en) ist.

4. Erzeugnis nach irgendeinem der Ansprüche 1 - 3, wobei das Nikotin in irgendeiner Form ein Teil des Kaugummikerns oder von mindestens einem der Kaugummikerne ist.

5. Erzeugnis nach irgendeinem der Ansprüche 1 - 4, wobei die mindestens eine Überzugsschicht auf so eine Art gepuffert ist, dass sich bei der Verabreichung des Gummis an ein Subjekt der pH des Speichels des Subjektes um 0,3 - 4 pH Einheiten erhöht.

6. Erzeugnis nach Anspruch 5, wobei die mindestens eine Überzugsschicht auf so eine Art gepuffert ist, dass sich bei der Verabreichung des Gummis an ein Subjekt der pH des Speichels des Subjektes um 0,5 - 2 pH Einheiten erhöht.

7. Erzeugnis nach irgendeinem der Ansprüche 1 - 6, wobei die mindestens eine Überzugsschicht durch die Verwendung eines Puffers gepuffert ist, der aus der Gruppe bestehend aus einem Carbonat, wie Monocarbonat, Bicarbonat oder Sesquicarbonat, Glycinat, Phosphat, Glycerophosphat, Acetat, Gluconat oder Citrat eines Alkalimetalls, wie Kalium oder Natrium, oder Ammonium, oder Mischungen davon ausgewählt ist, wobei optional der Puffer mikroverkapselt ist, und wobei die optionale Pufferung des mindestens einen Kaugummikerns durch die Verwendung eines Puffers nach der oberen Auswahl erhältlich ist.

8. Erzeugnis nach irgendeinem der Ansprüche 1 - 7, wobei das Nikotin in irgendeiner Form aus der Gruppe bestehend aus einem Nikotinsalz, Nikotin in Form der freien Base, einem Nikotinderivat, wie einem Nikotinkationaustauscher, einem Nikotineinschlusskomplex oder in irgendeiner Art nicht kovalent gebundenem Nikotin; Zeoltih gebundenem Nikotin, Cellulose oder Stärkemikrosphären gebundenem Nikotin oder Mischungen davon ausgewählt ist.

9. Erzeugnis nach Anspruch 8, wobei der Nikotineinschlusskomplex ein Cyklodextrinkomplex, wie β-Cyclodextrin, ist.

10. Erzeugnis nach Anspruch 8, wobei der Nikotinkationaustauscher ein Polyacrylatkationaustauscher ist.

11. Erzeugnis nach Anspruch 8, wobei das Nikotinsalz ein Salz ist das mit Tartrat, Citrat oder Malat gebildet wird.

12. Erzeugnis nach irgendeinem der Ansprüche 1-11, wobei das Nikotin in irgendeiner Form in einer Menge, berechnet als Nikotin in Form der freien Base pro Stück überzogenes Kaugummierzeugnis, von 0,05 - 10 mg vorliegt.

13. Erzeugnis nach Anspruch 12, wobei das Nikotin in irgendeiner Form in einer Menge, berechnet als Nikotin in Form der freien Base pro Stück überzogenes Kaugummierzeugnis, von 0,5 - 6 mg vorliegt.

14. Erzeugnis nach Anspruch 13, wobei das Nikotin in irgendeiner Form in einer Menge, berechnet als Nikotin in Form der freien Base pro Stück überzogenes Kaugummierzeugnis, von 2 - 5 mg vorliegt.

15. Erzeugnis nach irgendeinem der Ansprüche 1 - 14, wobei das Nikotin in irgendeiner Form in einer Menge, berechnet als Nikotin in Form der freien Base in mindestens einer der mindestens einen Überzugsschicht(en), von 0 - 10 mg vorliegt.

16. Erzeugnis nach Anspruch 15, wobei das Nikotin in irgendeiner Form in einer Menge, berechnet als Nikotin in Form der freien Base in mindestens einer der mindestens einen Überzugsschicht(en), von 0,2 - 6 mg vorliegt.

17. Erzeugnis nach Anspruch 16, wobei das Nikotin in irgendeiner Form in einer Menge, berechnet als Nikotin in Form der freien Base in mindestens einer der mindestens einen Überzugsschicht(en), von 0,5 - 5 mg vorliegt.

18. Erzeugnis nach irgendeinem der Ansprüche 1 - 17, wobei das Nikotin nachahmende Agens ein Agens mit stechend brennendem Geschmack ist.

19. Erzeugnis nach Anspruch 18, wobei das Nikotin nachahmende Agens ausgewählt aus Capsaicin, Piperin und Zingeron oder einer Mischung davon ist.

20. Erzeugnis nach irgendeinem der Ansprüche 1 - 19, wobei das/die optionale(n) mindestens eine oder mehreren Additiv(e) aus der Gruppe bestehend aus Stabilisatoren, wie Konservierungsmitteln, z. B. Antioxidantien; Weichmachern, Verdickern, Emulgatoren, Gleitmitteln, Schmiermitteln, Süßstoffen, Geschmacksstoffen, Aromastoffen, Verstärkern, Färbungsmitteln, Vitaminen, Mineralien und Mischungen davon ausgewählt ist/sind.

21. Erzeugnis nach irgendeinem der Ansprüche 1 - 20, wobei der Überzug, besonders wenn ein Zuckerüberzug benutzt wird, das Nikotin, bevorzugt in der Form von Nikotinhydrogentartrat (NHT), und der Puffer voneinander durch das Aufbewahren in separaten Schichten getrennt sind, wobei die genannten Schichten optional durch eine Feuchtigkeitsbarriere separiert sind, wobei die genannte Feuchtigkeitsbarriere Substanzen umfasst, die aus Ethylcellulose, Hydroxpropylmethylcellulose und Polymethacrylaten oder Kombinationen davon, bevorzugt kombiniert mit einem oder mehreren Weichmachern und/oder hydrophoben, lipid-basierenden Filmen, wie Filmen, die Stearinsäure umfassen, ausgewählt sind.

22. System zur Zuführung von Nikotin in irgendeiner Form an ein Subjekt, umfassend ein überzogenes Kaugummierzeugnis nach irgendeinem der Ansprüche 1 - 21 und mindestens ein anderes Mittel oder Verfahren, um eine Verminderung des Drangs zu Rauchen oder Tabak zu benutzen zu erhalten.

23. System, um eine Verminderung des Drangs zu Rauchen oder Tabak zu benutzen zu erhalten und/oder um ein Gefühl der Befriedigung durch Rauchen ohne Rauchen zur Verfügung zu stellen, umfassend ein überzogenes Kaugummierzeugnis nach den Ansprüchen 1 - 21 und mindestens ein anderes Mittel oder Verfahren, um eine Verminderung des Drangs zu Rauchen oder Tabak zu benutzen zu erhalten.

24. System nach den Ansprüchen 22 oder 23, wobei das mindestens eine andere Mittel oder Verfahren ein Begleit- oder konkurrierendes Mittel oder Verfahren ist, das aus der Gruppe bestehend aus der Verabreichung durch Mundsprays, Nasensprays, transdermalen Pflastern, Inhalationsvorrichtungs, Lutschtabletten, Tabletten und parenteralen Verfahren, subkutanen Verfahren, intravenösen Verfahren, rektalen Verfahren, vaginalen Verfahren und transmukosalen Verfahren, oder der Verwendung von Tabak ausgewählt ist.

25. Verfahren nach Anspruch 24, wobei das mindestens andere Mittel oder Verfahren die Verabreichung von Nikotin umfasst.

26. Verfahren für die Herstellung eines beschichteten Kaugummierzeugnisses nach den Ansprüchen 1 - 21, umfassend die Schritte
a) zur Verfügung stellen von mindestens einem Kaugummikern und/oder zur Verfügung stellen von mindestens einem Nikotin beinhaltenden Kaugummikern
b) zur Verfügung stellen von Nikotin in irgendeiner Form,
c) zur Verfügung stellen von mindestens einem Überzug, der gepuffert ist,
d) Hinzufügen des Nikotins in irgendeiner Form zu dem mindestens einen Kaugummikern und/oder zu dem mindestens einen Überzug, und
e) Überziehen des mindestens einen Kaugummikerns mit mindestens einer Schicht der mindestens einen gepufferten Überzugsschicht.

27. Verfahren nach Anspruch 26 weiterhin umfassend die Schritte
f) Puffern des mindestens einen Kaugummikerns, und/oder
g) zur Verfügung stellen von mindestens einer Schicht, die nicht gepuffert ist, und optional
h) Hinzufügen von Nikotin in irgendeiner Form zu mindestens einer der genannten mindestens einen Überzugsschicht, die nicht gepuffert ist, und optional
i) zur Verfügung stellen von Nikotin in dem Überzug und des Puffers in dem Überzug in separaten Schichten, bevorzugt separiert durch eine Feuchtigkeitsbarriere.

28. Verfahren nach irgendeinem der Ansprüche 26 - 27, wobei das Nikotin in irgendeiner Form aus der Gruppe bestehend aus einem Nikotinsalz, Nikotin in Form der freien Base, einem Nikotinderivat, wie einem Nikotinkationaustauscher, einem Nikotineinschlusskomplex oder in irgendeiner Art nicht kovalent gebundenem Nikotin; Zeoltih gebundenem Nikotin, Cellulose oder Stärkemikrosphären gebundenem Nikotin oder Mischungen davon ausgewählt ist.

29. Verfahren nach irgendeinem der Ansprüche 26 - 28, wobei die mindestens eine Überzugsschicht durch die Verwendung eines Puffers gepuffert ist, der aus der Gruppe bestehend aus einem Carbonat, wie Monocarbonat, Bicarbonat oder Sesquicarbonat, Glycinat, Phosphat, Glycerophosphat, Acetat, Gluconat oder Citrat eines Alkalimetalls, wie Kalium oder Natrium, oder Ammonium, oder Mischungen davon ausgewählt ist, wobei die mindestens eine Überzugsschicht auf so eine Art gepuffert ist, dass sich bei der Verabreichung des Gummis an ein Subjekt der pH des Speichels des Subjekts um 0,3 - 4 pH Einheiten erhöht.

30. Verfahren nach Anspruch 29, wobei die mindestens eine Überzugsschicht auf so eine Art gepuffert ist, dass sich bei der Verabreichung des Gummis an ein Subjekt der pH des Speichels des Subjektes um 0,5 - 2 pH Einheiten erhöht.

31. Verfahren nach irgendeinem der Ansprüche 26 - 30, wobei das zur Verfügungstellen des mindestens einen Kaugummikerns in Schritt a) die Schritte umfasst
a1) zur Verfügung stellen eines Gummikernteigs,
a2) Mischen, Walzen und Falzen; Giessen; oder Extrudieren des Gummiteigs.

32. Verfahren nach irgendeinem der Ansprüche 26 - 30, wobei das zur Verfügung stellen des Kaugummikerns in Schritt a) erhältlich ist durch direktes Zusammenpressen der Bestandteile.

33. Verfahren nach irgendeinem der Ansprüche 26 - 32, wobei das Überziehen des mindestens einen Kaugummikerns mit mindestens einer Schicht des mindestens einen gepufferten Überzugs die Schritte umfasst
a) Filmüberziehen, und/oder
b) Pressüberziehen, und/oder
c) Zuckerüberziehen, und/oder
d) Schmelzüberziehen.

34. Kaugummierzeugnis nach irgendeinem der Ansprüche 1 - 21 zur therapeutische Verwendung.

35. Erzeugnis nach Anspruch 34, wobei die Therapie die Behandlung einer Krankheit ist, die aus der Gruppe bestehend aus Alzheimer, Morbus Crohn, Parkinson, Tourette Syndrom, eiternder Kolitis und Postrauchentwöhnungs-Gewichtskontrolle ausgewählt ist.

36. Verwendung von Nikotin für die Herstellung eines Kaugummierzeugnisses nach irgendeinem der Ansprüche 1 - 21 für die Behandlung einer Krankheit, die aus der Gruppe bestehend aus Alzheimer, Morbus Crohn, Parkinson, Tourette Syndrom, eiternder Kolitis und Postrauchentwöhnungs-Gewichtskontrolle ausgewählt ist.

37. Verwendung eines Kaugummis für die Herstellung eines Nikotin beinhaltenden Kaugummierzeugnisses nach irgendeinem der Ansprüche 1 - 21 für die Behandlung einer Krankheit, die aus der Gruppe bestehend aus Alzheimer, Morbus Crohn, Parkinson, Tourette Syndrom, eiternder Kolitis und Postrauchentwöhnungs-Gewichtskontrolle ausgewählt ist.

## Revendications

1. Produit de chewing-gum enrobé comprenant au moins un noyau de chewing-gum tamponné ou non-tamponné, de la nicotine sous n'importe quelle forme et/ou un agent imitant la nicotine, au moins une couche de revêtement et éventuellement un ou plusieurs autres additifs, dans lequel ladite au moins une couche de revêtement est tamponnée.

2. Produit selon la revendication 1, dans lequel ledit au moins un noyau est tamponné.

3. Produit selon l'une quelconque des revendications 1-2, dans lequel la nicotine sous n'importe quelle forme fait partie de ladite au moins une couche de revêtement ou d'au moins une desdites au moins une couche de revêtement.

4. Produit selon l'une quelconque des revendications 1-3, dans lequel la nicotine sous n'importe quelle forme, fait partie du noyau de chewing-gum ou d'au moins un noyau de chewing-gum.

5. Produit selon l'une quelconque des revendications 1-4, dans lequel ladite au moins une couche de revêtement est tamponnée de sorte que, lors de l'administration de la gomme à un sujet, le pH de la salive du sujet est augmenté de 0,3 - 4 unités de pH.

6. Produit selon la revendication 5, dans lequel ladite au moins une couche de revêtement est tamponnée de sorte que, lors de l'administration de la gomme à un sujet, le pH de la salive du sujet est augmenté par 0,5 - 2 unités de pH.

7. Produit selon l'une quelconque des revendications 1-6, dans lequel ladite au moins une couche de revêtement est tamponnée par l'utilisation d'un tampon choisi dans le groupe constitué d'un carbonate, comme un monocarbonate, un bicarbonate ou un sesquicarbonate, un glycinate, un phosphate, un glycérophosphate, un acétate, un gluconate ou un citrate d'un métal alcalin, comme le potassium ou le sodium, ou l'ammonium et des mélanges de ceux-ci, moyennant quoi, éventuellement, le tampon est micro-encapsulé, et dans lequel le tamponnage éventuel dudit au moins un noyau de chewing-gum est obtenu en utilisant un tampon selon la sélection ci-dessus.

8. Produit selon l'une quelconque des revendications 1-7, dans lequel la nicotine, sous n'importe quelle forme, est choisie dans le groupe constitué d'un sel de nicotine, la forme de base libre de la nicotine, un dérivé de nicotine, comme un échangeur de cation de nicotine, un complexe d'inclusion de nicotine, ou de la nicotine dans n'importe quelle liaison non-covalente ; la nicotine liée aux zéolites ; la nicotine liée à la cellulose ou aux micro-billes d'amidon ; et des mélanges de ceux-ci.

9. Produit selon la revendication 8, dans lequel le complexe d'inclusion de nicotine est un complexe de cyclodextrine, comme la β-cyclodextrine.

10. Produit selon la revendication 8, dans lequel l'échangeur de cation de nicotine est un échangeur de cation de polyacrylate.

11. Produit selon la revendication 8, dans lequel le sel de nicotine est un sel formé avec du tartrate, du citrate ou du malate.

12. Produit selon l'une quelconque des revendications 1-11, dans lequel la nicotine sous n'importe quelle forme est présente dans une quantité de 0,05-10 mg, calculée comme base libre de nicotine par pièce de produit de chewing-gum revêtu.

13. Produit selon la revendication 12, dans lequel la nicotine sous n'importe quelle forme est présente dans une quantité de 0,5-6 mg calculé comme la forme de base libre de nicotine par pièce de produit de chewing-gum revêtu.

14. Produit selon la revendication 13, dans lequel la nicotine sous n'importe quelle forme est présente dans une quantité de 2-5 mg calculée comme la forme de base libre de la nicotine par pièce de produit de chewing-gum revêtu.

15. Produit selon l'une quelconque des revendications 1-14, dans lequel la nicotine sous n'importe quelle forme est dans une quantité de 0-10 mg, calculée comme la forme de base libre de la nicotine, dans au moins une des couches de revêtement.

16. Produit selon la revendication 15, dans lequel la nicotine sous n'importe quelle forme est dans une quantité de 0,2 - 6 mg, calculée comme la forme de base libre de nicotine dans au moins un des couches de revêtement.

17. Produit selon la revendication 16, dans lequel la nicotine sous n'importe quelle forme est dans une quantité de 0,5 - 5 mg calculée comme la forme de base libre de nicotine dans au moins une des couches de revêtement.

18. Produit selon l'une quelconque des revendications 1-17, dans lequel l'agent imitant la nicotine est n'importe quel agent avec un goût de brûlé âcre.

19. Produit selon la revendication 18, dans lequel l'agent de simulation de nicotine est choisi parmi la capsaïcine, la pipérine et la zingérone ou tout mélange de ceux-ci.

20. Produit selon l'une quelconque des revendications 1-19, dans lequel ledit au moins un ou plusieurs additifs est choisi dans le groupe constitué d'agents stabilisants, comme des conservateurs, par exemple des antioxydants ; des adoucissants, des agents épaississants, des émulsionnants, des glissants, des lubrifiants, des édulcorants, des parfums, des arômes, des exhausteurs, des agents colorants, des vitamines, des minéraux, et des mélanges de ceux-ci.

21. Produit selon l'une quelconque des revendications 1-20, dans lequel dans le revêtement, en particulier quand un revêtement en sucre est utilisé, la nicotine, de préférence sous la forme de tartrate d'hydrogène de nicotine (NHT) et le tampon sont séparés l'un de l'autre en étant maintenus dans des couches séparées, moyennant quoi lesdites couches sont éventuellement séparées par une barrière d'humidité, ladite barrière d'humidité comprenant des substances choisies parmi l'éthylcellulose, l'hydroxypropylméthylcellulose, et les polyméthacrylates ou des combinaisons de ceux-ci, de préférence combinés avec un ou plusieurs plastifiants et/ou des films à base de lipide hydrophobe comme des films comprenant de l'acide stéarique.

22. Système de distribution de nicotine sous n'importe quelle forme à un sujet, comprenant un produit de chewing-gum revêtu selon l'une quelconque des revendications 1-21, et au moins un autre moyen ou procédé pour obtenir une réduction de l'envie de fumer ou d'utiliser.

23. Système pour obtenir une réduction de l'envie de fumer ou d'utiliser du tabac et/ou pour fournir une sensation de satisfaction de fumer sans fumer, comprenant un produit de chewing-gum revêtu selon la revendication 1-21, et au moins un autre moyen ou procédé pour obtenir la réduction de l'envie de fumer ou d'utiliser du tabac.

24. Système selon la revendication 22 ou 23, dans lequel ledit au moins un autre moyen ou procédé est un moyen ou procédé simultané ou concomitant choisi dans le groupe constitué d'une administration par des sprays buccaux, des sprays nasaux, des patchs transdermiques, des dispositifs inhalation, des comprimés, des cachets et des procédés parentéraux, des procédés sous-cutanés, des procédés intraveineux, des procédés rectaux, des procédés vaginaux, et des procédés transmuqueux, ou l'utilisation de tabac.

25. Système selon la revendication 24, dans lequel ledit au moins un autre moyen ou procédé comprend l'administration de nicotine.

26. Procédé de production d'un produit de chewing-gum revêtu selon la revendication 1-21, comprenant les étapes consistant à :
a) fournir au moins un noyau de chewing-gum, et/ou fournir au moins un noyau de chewing-gum contenant de la nicotine;
b) fournir de la nicotine sous n'importe forme ;
c) fournir au moins un revêtement qui est tamponné ;
d) ajouter la nicotine sous n'importe quelle forme audit au moins un noyau de chewing-gum et/ou audit au moins un revêtement, et
e) revêtir ledit au moins un noyau de chewing-gum avec au moins une couche de ladite au moins couche de revêtement tamponnée.

27. Procédé selon la revendication 26, comprenant en outre les étapes consistant à :
f) tamponner ledit au moins un noyau de chewing-gum, et/ou
g) fournir au moins une couche de revêtement non tamponnée, et éventuellement
h) ajouter la nicotine sous n'importe quelle forme à au moins une desdites au moins une couche de revêtement non tamponnée, et éventuellement
i) fournir la nicotine dans le revêtement et le tampon dans le revêtement dans des couches séparées, de préférence séparées par une barrière d'humidité.

28. Procédé selon l'une quelconque des revendications 26-27, dans lequel la nicotine, sous n'importe quelle forme, est choisie dans le groupe constitué d'un sel de nicotine, la nicotine sou forme de base libre, un dérivé de nicotine, comme un échangeur de cation de nicotine, un complexe d'inclusion de nicotine, ou de la nicotine sous n'importe quelle liaison non-covalente ; la nicotine liée aux zéolites ; la nicotine liée à la cellulose ou des microbilles d'amidon ; et des mélanges de ceux-ci.

29. Procédé selon l'une quelconque des revendications 26-28, dans lequel ledit au moins une couche de revêtement est tamponnée par l'utilisation d'un tampon choisi dans le groupe constitué d'un carbonate comprenant du bicarbonate ou sesquicarbonate, du glycinate, du phosphate, du glycérophosphate, ou du citrate d'un métal alcalin, comme le potassium ou le sodium, ou l'amonium, et des mélanges de ceux-ci, dans lequel la au moins une couche de revêtement est tamponée de sorte que, lors de l'administration de la gomme à un sujet, le pH de la salive du sujet est augmenté de 0,3-4 unités de pH.

30. Procédé selon la revendication 29, dans lequel ladite au moins une couche de revêtement est tamponnée de sorte que, lors de l'administration de la gomme à un sujet, le pH de la salive du sujet est augmentée de 0,5-2 unités de pH.

31. Procédé selon l'une quelconque des revendications 26-30, dans lequel la fourniture dudit au moins un noyau de chewing-gum dans l'étape a) comprend les étapes consistant à
a1) fournir une pâte de noyau de gomme,
a2) mélanger, rouler et entailler ; mouler ou extruder la pâte de gomme.

32. Procédé selon l'une quelconque des revendications 26-30, dans lequel la fourniture du noyau de chewing-gum dans l'étape a) est obtenue par compression directe des ingrédients.

33. Procédé selon l'une quelconque des revendications 26-32, dans lequel le revêtement dudit au moins un noyau de chewing-gum avec au moins une couche dudit au moins un revêtement tamponné comprend les étapes consistant à :
a) un pelliculage, et/ou
b) un enrobage à sec, et/ou
c) une dragéification, et/ou
d) couchage par fusion.

34. Produit de chewing-gum selon l'une quelconque des revendications 1-21 à utiliser en thérapie.

35. Produit selon la revendication 34, dans lequel la thérapie est un traitement d'une maladie choisie dans le groupe constitué de la maladie d'Alzheimer, la maladie de Crohn, la maladie de Parkinson, le syndrome de Tourette, la rectocolite hémorragique et le contrôle du poids après l'arrêt de la cigarette.

36. Utilisation de nicotine pour la production d'un chewing-gum selon l'une quelconque des revendications 1-21 pour le traitement d'une maladie choisie dans le groupe constitué de la maladie d'Alzheimer, la maladie de Crohn, la maladie de Parkinson, le syndrome de Tourette, la rectocolite hémorragique et le contrôle du poids après l'arrêt de la cigarette.

37. Utilisation d'un chewing-gum pour la production d'un produit de chewing-gum contenant de la nicotine selon l'une quelconque des revendications 1-21, pour le traitement d'une maladie choisie dans le groupe constitué de la maladie d'Alzheimer, la maladie de Crohn, la maladie de Parkinson, le syndrome de Tourette, la rectocolite hémorragique et le contrôle du poids après l'arrêt de la cigarette.
